(19)

Europäisches
Patentamt
European
Patent Office
Office européen
des brevets

(11)  **EP 4 759 941 A1**

(12)  **EUROPEAN PATENT APPLICATION**

(43) Date of publication:
**17.06.2026  Bulletin 2026/25**

(21) Application number: **24219335.7**

(22) Date of filing: **12.12.2024**

(51) International Patent Classification (IPC):
***C12Q 1/6886*** *(2018.01)*

(52) Cooperative Patent Classification (CPC):
**C12Q 1/6886;** C12Q 2600/106; C12Q 2600/118;
C12Q 2600/158

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AE Eindhoven (NL)**

(72) Inventor: **HOFFMANN, Ralf Dieter
Eindhoven (NL)**

(74) Representative: **Philips Intellectual Property &
Standards
High Tech Campus 34
5656 AE Eindhoven (NL)**

<u>Remarks:</u>
The complete document including Reference
Table(s) and the Sequence Listing(s) can be
downloaded from the EPO website

(54)  **PREDICTING THE RESPONSE OF A PATIENT TO A TREATMENT FOR BLADDER CANCER**

(57)   The invention relates to methods for determining effectiveness of a FGFR inhibitor in the treatment of bladder cancer in a subject. The invention is based on determining the expression level of one or multiple marker genes which were found indicative of FGFR inhibitor sensitivity. The invention further describes FGFR inhibitors for use in the treatment of bladder cancer when the cancer is likely to respond to the FGFR inhibitor. Further described are kits and uses thereof for use in the methods described herein.

y = 3.614 + -0.155 x
n = 17
r = 0.64; P = 0.005

**Fig. 4**

**Description**

FIELD OF THE INVENTION

The invention relates to methods for determining effectiveness of a FGFR inhibitor in the treatment of bladder cancer in a subject. The invention is based on determining the expression level of one or multiple marker genes which were found indicative of FGFR inhibitor sensitivity. The invention further describes FGFR inhibitors for use in the treatment of bladder cancer when the cancer is likely to respond to the FGFR inhibitor. Further described are kits and uses thereof for use in the methods described herein.

BACKGROUND OF THE INVENTION

Fibroblast Growth Factor Receptors (FGFRs) are a family of receptor tyrosine kinases that play essential roles in regulating cell proliferation, differentiation, survival, and angiogenesis. The FGFR family, comprising FGFR1, FGFR2, FGFR3, and FGFR4, interacts with specific fibroblast growth factors (FGFs) to initiate signaling cascades. These pathways, including the MAPK, PI3K-AKT, and JAK-STAT pathways, govern various cellular processes, making FGFRs integral to both normal cellular function and pathological conditions, such as cancer. In bladder cancer, FGFR alterations have garnered significant attention, particularly in the context of non-muscle invasive bladder cancer (NMIBC), where FGFR3 mutations and fusions are highly prevalent.

Bladder cancer is a heterogenous disease categorized into non-muscle invasive and muscle-invasive forms. NMIBC constitutes a significant proportion of cases, and while it is associated with better survival outcomes compared to muscle-invasive bladder cancer (MIBC), it carries substantial risks of recurrence and progression. FGFR3 mutations are observed in approximately 50-70% of NMIBC cases, particularly in low-grade and papillary subtypes. This prevalence underscores the critical role of FGFR3 in the biology of NMIBC. Common FGFR3 mutations, such as S249C, R248C, and Y373C, result in constitutive activation of the receptor, bypassing normal regulatory mechanisms. FGFR3 gene fusions, including FGFR3-TACC3, further amplify its oncogenic potential by enhancing dimerization and sustained signaling. These alterations drive tumorigenesis through pathways that promote cell proliferation, inhibit apoptosis, and stimulate angiogenesis.

The impact of FGFR3 alterations on NMIBC progression is nuanced. While these mutations are more common in low-grade, non-invasive tumors with a favorable prognosis, their presence in recurrent tumors highlights their role in disease persistence. FGFR3 mutations may also interact with other oncogenic pathways, such as alterations in TP53 or PI3K, to contribute to disease progression. Despite being associated with less aggressive tumors initially, these alterations may create a foundation for more advanced disease when coupled with additional genetic or epigenetic changes.

The clinical relevance of FGFR3 in NMIBC extends beyond its role in disease biology. FGFR3 mutations serve as biomarkers for prognosis and therapeutic intervention. Patients with FGFR3-mutant tumors typically have a more favorable prognosis due to the association with low-grade disease; however, these mutations are also implicated in higher rates of recurrence. This dual role underscores the need for targeted therapeutic strategies to manage FGFR3-driven tumors effectively.

Targeted therapies against FGFRs have emerged as a promising avenue in bladder cancer treatment, particularly in cases where conventional therapies such as transurethral resection of bladder tumor (TURBT) or intravesical Bacillus Calmette-Guérin (BCG) fail. FGFR inhibitors, which include both small molecule inhibitors and monoclonal antibodies, aim to block the aberrant signaling caused by FGFR alterations. Among these, erdafitinib, a pan-FGFR inhibitor, has shown substantial efficacy in clinical trials for bladder cancer, particularly in patients with FGFR3-mutant or FGFR3-fusion tumors. While currently approved for advanced or metastatic bladder cancer, its application in NMIBC is being actively explored.

In NMIBC, FGFR inhibitors present an opportunity to address high-risk and recurrent disease. Erdafitinib has demonstrated activity against FGFR3-driven tumors, making it a candidate for treatment in cases resistant to intravesical therapies such as BCG. Another promising agent, infigratinib (BGJ398), is under investigation for its potential to treat FGFR3-mutant NMIBC. These therapies work by selectively targeting FGFR3 and interrupting its downstream signaling, thereby inhibiting tumor growth and promoting apoptosis.

While FGFR-targeted therapies are promising, several challenges must be addressed to optimize their efficacy. The heterogeneity of FGFR3 mutations and fusions means that not all alterations have equivalent oncogenic potential, and their impact on therapy response varies. Moreover, resistance to FGFR inhibitors, often through secondary mutations or activation of alternative pathways such as MET or VEGFR, poses a significant hurdle. Overcoming these resistance mechanisms requires innovative approaches, including combination therapies. Combining FGFR inhibitors with immune checkpoint inhibitors or chemotherapeutic agents is an area of active research, aiming to leverage the synergistic effects of targeting multiple pathways.

Patient selection is another critical factor in the success of FGFR-targeted therapies. Molecular profiling to identify patients with FGFR3 alterations is essential for ensuring that these therapies are administered to those most likely to benefit. This precision medicine approach not only enhances treatment efficacy but also minimizes unnecessary exposure to potentially toxic therapies in patients unlikely to respond.

Integrating FGFR-targeted therapies into the standard care for NMIBC requires careful consideration. The role of these therapies in combination with or as alternatives to established treatments, such as TURBT, BCG, and intravesical chemotherapy, must be clearly defined. Furthermore, the potential of intravesical delivery of FGFR inhibitors offers an exciting avenue for localized treatment, which could achieve high drug concentrations in the bladder while minimizing systemic side effects.

The ongoing evolution of FGFR-targeted therapies is supported by numerous clinical trials that aim to establish their efficacy and safety in NMIBC. These studies are expected to provide critical insights into optimal dosing, delivery methods, and combination strategies. Additionally, they will help clarify the long-term benefits of these therapies in reducing recurrence and preventing progression to muscle-invasive disease.

In conclusion, FGFR, particularly FGFR3, plays a pivotal role in the progression and recurrence of NMIBC. Its alterations not only drive tumorigenesis but also present a valuable target for therapeutic intervention. The development of FGFR inhibitors represents a significant advancement in the management of bladder cancer, offering hope for improved outcomes in high-risk and refractory cases. However, optimizing their use requires addressing challenges such as resistance, patient selection, and integration with existing treatment paradigms. Through continued research and clinical innovation, FGFR-targeted therapies have the potential to transform the treatment landscape for NMIBC, improving both survival and quality of life for patients

These drawbacks, among others, are overcome by the products and methods as outlined in the appended claims.

SUMMARY OF THE INVENTION

In a first aspect the invention relates to a Fibroblast Growth Factor Receptor (FGFR) inhibitor for use in the treatment of bladder cancer in a subject in need thereof, the use comprising determining in a bladder cancer sample obtained from the subject the expression level of PDE4D, and administering the FGFR inhibitor to the subject if the expression levels is above a predetermined threshold.

In a second aspect the invention relates to a Fibroblast Growth Factor Receptor (FGFR) inhibitor for use in the treatment of bladder cancer in a subject in need thereof, the use comprising determining in a bladder cancer sample obtained from the subject the expression levels of three or more genes selected from APOBEC3A, CIAO1, DDX58, DHX9, IFI16, IFIH1, LRRFIP1, OAS1, ZBP1, EZR, and PDE4D, and calculating a FGFR inhibitor treatment score based on the three or more expression levels, and administering the FGFR inhibitor to the subject if the FGFR inhibitor treatment score is below a predetermined threshold.

In a third aspect the invention relates to a computer implemented method of predicting effectiveness of treating a subject with bladder cancer with an FGFR inhibitor, the method comprising receiving the expression level of PDE4D determined in a bladder cancer sample obtained from the subject, and predicting the effectiveness of treating the subject with a FGFR inhibitor based on the PDE4D expression level.

In a fourth aspect the invention relates to a computer implemented method of predicting effectiveness of treating a subject with bladder cancer with an FGFR inhibitor, the method comprising receiving the expression levels of three or more genes selected from APOBEC3A, CIAO1, DDX58, DHX9, IFI16, IFIH1, LRRFIP1, OAS1, ZBP1, EZR, and PDE4D determined in a bladder cancer sample obtained from the subject, and calculating a FGFR inhibitor treatment score based on the three or more expression levels, and predicting he effectiveness of treating the subject with a FGFR inhibitor based on the FGFR inhibitor treatment score.

In a fifth aspect the invention relates to a kit of parts kit of parts comprising a FGFR inhibitor and means for determining the expression levels of PDE4D and/or three or more genes selected from APOBEC3A, CIAO1, DDX58, DHX9, IFI16, IFIH1, LRRFIP1, OAS1, ZBP1, EZR, and PDE4D.

In a sixth aspect the invention relates to the use of a kit to predict effectiveness of treating a subject with bladder cancer with an FGFR inhibitor, the kit comprising means for determining the expression levels of PDE4D and/or three or more genes selected from APOBEC3A, CIAO1, DDX58, DHX9, IFI16, IFIH1, LRRFIP1, OAS1, ZBP1, EZR, and PDE4D in a sample, wherein the use comprises determining the expression levels of PDE4D and/or three or more genes selected from APOBEC3A, CIAO1, DDX58, DHX9, IFI16, IFIH1, LRRFIP1, OAS1, ZBP1, EZR, and PDE4D in a bladder cancer sample from a subject with bladder cancer, and predicting the effectiveness of treating the subject with bladder cancer with an FGFR inhibitor based on the PDE4D expression level and/or the expression levels of the three or more genes selected from APOBEC3A, CIAO1, DDX58, DHX9, IFI16, IFIH1, LRRFIP1, OAS1, ZBP1, EZR, and PDE4D.

BRIEF DESCRIPTION OF THE DRAWINGS

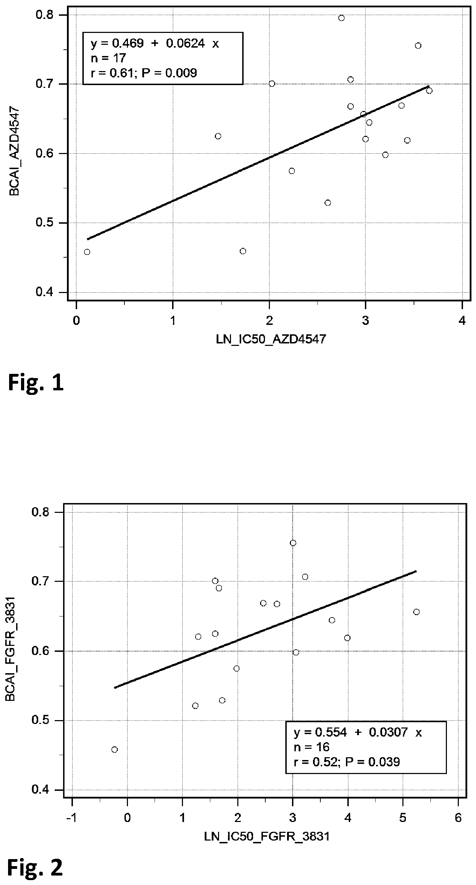

Fig. 1: depicts 17 bladder cancer cell lines were tested in vitro for their sensitivity to the FGFR inhibitor (FGFRi) AZD4547. Multiple doses per cell line were tested and the IC50 value of the dose with 50% inhibition of proliferation was determined. In parallel, for each cell line a gene expression profile was created based on cellular RNA run on a DNA microarray. For each cell line expressed gene a quantified expression value was determined. Based on the quantified expression values the BCAI ImmunoScore was calculated. Finally, linear regression analysis of the LN (natural log) of the IC50 value of AZD4547 vs the LOG2 value of the bladder cancer BCAI ImmunoScore as was performed. Each circle represents one cell line. The parameters of the regression line (intercept and slope) are given along with the correlation coefficient r and the p-value for the goodness of the fit of the regression line.

Fig. 2 depicts 16 bladder cancer cell lines were tested in vitro for their sensitivity to the FGFR inhibitor (FGFRi) FGFR_3831. Multiple doses per cell line were tested and the IC50 value of the dose with 50% inhibition of proliferation was determined. In parallel, for each cell line a gene expression profile was created based on cellular RNA run on a DNA microarray. For each cell line expressed gene a quantified expression value was determined. Based on the quantified expression values the BCAI ImmunoScore was calculated. Finally, linear regression analysis of the LN (natural log) of the IC50 value of FGFR 3831 vs the LOG2 value of the bladder cancer BCAI ImmunoScore as was performed. Each circle represents one cell line. The parameters of the regression line (intercept and slope) are given along with the correlation coefficient r and the p-value for the goodness of the fit of the regression line.

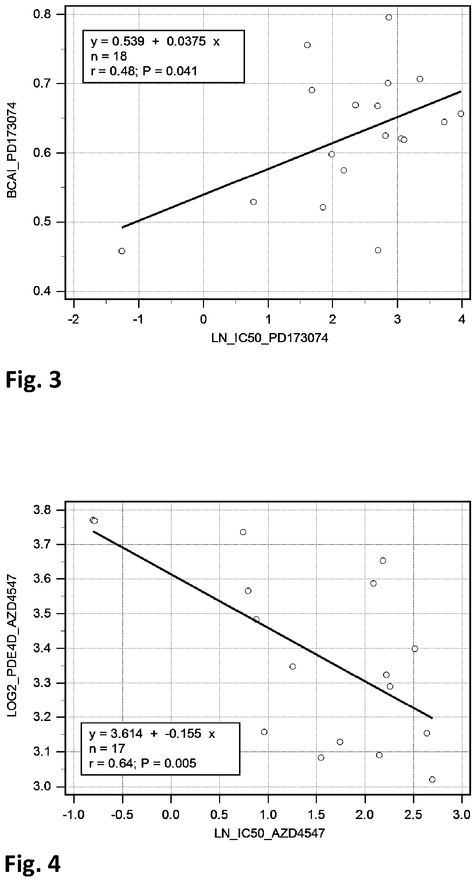

Fig. 3 depicts 18 bladder cancer cell lines were tested in vitro for their sensitivity to the FGFR inhibitor (FGFRi) PD173074. Multiple doses per cell line were tested and the IC50 value of the dose with 50% inhibition of proliferation was determined. In parallel, for each cell line a gene expression profile was created based on cellular RNA run on a DNA microarray. For each cell line expressed gene a quantified expression value was determined. Based on the quantified expression values the BCAI ImmunoScore was calculated. Finally, linear regression analysis of the LN (natural log) of the IC50 value of PD173074 vs the LOG2 value of the bladder cancer BCAI ImmunoScore as was performed. Each circle represents one cell line. The parameters of the regression line (intercept and slope) are given along with the correlation coefficient r and the p-value for the goodness of the fit of the regression line.

Fig. 4 depicts 17 bladder cancer cell lines were tested in vitro for their sensitivity to the FGFR inhibitor (FGFRi) AZD4547. Multiple doses per cell line were tested and the IC50 value of the dose with 50% inhibition of proliferation was determined. In parallel, for each cell line a gene expression profile was created based on cellular RNA run on a DNA microarray. For each cell line expressed gene a quantified expression value was determined. Finally, linear regression analysis of the LN (natural log) of the IC50 value of AZD4547 vs the LOG2 value of the PDE4D gene expression was performed. Each circle represents one cell line. The parameters of the regression line (intercept and slope) are given along with the correlation coefficient r and the p-value for the goodness of the fit of the regression line.

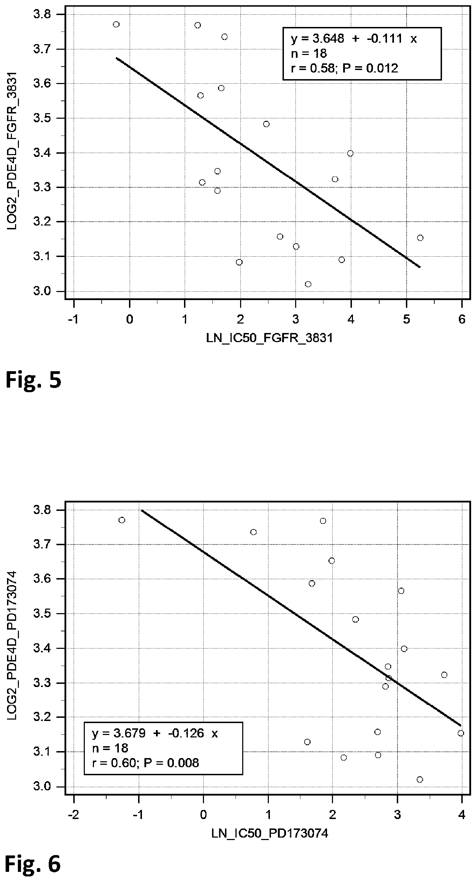

Fig. 5 depicts 16 bladder cancer cell lines were tested in vitro for their sensitivity to the FGFR inhibitor (FGFRi) FGFR_3831. Multiple doses per cell line were tested and the IC50 value of the dose with 50% inhibition of proliferation was determined.

In parallel, for each cell line a gene expression profile was created based on cellular RNA run on a DNA microarray. For each cell line expressed gene a quantified expression value was determined.

Finally, linear regression analysis of the LN (natural log) of the IC50 value of AZD4547 vs the LOG2 value of the PDE4D gene expression was performed. Each circle represents one cell line.

The parameters of the regression line (intercept and slope) are given along with the correlation coefficient r and the p-value for the goodness of the fit of the regression line.

Fig. 6 depicts 18 bladder cancer cell lines were tested in vitro for their sensitivity to the FGFR inhibitor (FGFRi) PD173074. Multiple doses per cell line were tested and the IC50 value of the dose with 50% inhibition of proliferation was determined. In parallel, for each cell line a gene expression profile was created based on cellular RNA run on a DNA microarray. For each cell line expressed gene a quantified expression value was determined. Finally, linear regression analysis of the LN (natural log) of the IC50 value of AZD4547 vs the LOG2 value of the PDE4D gene expression was performed. Each circle represents one cell line. The parameters of the regression line (intercept and slope) are given along with the correlation coefficient r and the p-value for the goodness of the fit of the regression line.

Definitions

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm 20\%$, preferably $\pm 15\%$, more preferably $\pm 10\%$, and even more preferably $\pm 5\%$.

"About" and "approximately": these terms, when referring to a measurable value such as an amount, a temporal duration, and the like, is meant to encompass variations of $\pm 20\%$ or $\pm 10\%$, more preferably $\pm 5\%$, even more preferably $\pm 1\%$, and still more preferably $\pm 0.1\%$ from the specified value, as such variations are appropriate to perform the disclosed methods.

"Antagonist" and "inhibitor": These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

"Anti-cancer effect": This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

"Alleviating cancer": The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

"Compositions", "products" or "combinations": These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

"Combination therapy", or "in combination with": These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of" and "consisting of".

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject.

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention. Unless otherwise defined, all technical and scientific terms used herein have the same meaning as commonly understood by a person of ordinary skill in the art.

Patient selection is a cornerstone of effective FGFR-targeted therapy in non-muscle invasive bladder cancer (NMIBC). This approach hinges on the understanding that FGFR inhibitors are not broadly effective for all patients but instead offer substantial benefits to those whose tumors exhibit specific genetic alterations. These alterations, predominantly mutations and fusions in the FGFR3 gene, drive oncogenic processes in a significant subset of NMIBC cases. Consequently, the identification and selection of patients with these genetic markers are essential for optimizing treatment outcomes, minimizing unnecessary exposure to treatment-associated toxicities, and ensuring the cost-effectiveness of FGFR inhibitors.

NMIBC is a heterogeneous disease, and its biological variability significantly influences treatment response. A defining characteristic of NMIBC associated with FGFR-targeted therapy is the high prevalence of FGFR3 mutations and fusions in certain subtypes. Studies have shown that FGFR3 alterations are present in approximately 50-70% of low-grade, papillary NMIBC cases. These tumors tend to exhibit a less aggressive clinical course and are heavily reliant on FGFR signaling pathways for proliferation and survival. By contrast, high-grade NMIBC and muscle-invasive bladder cancer (MIBC) demonstrate a lower frequency of FGFR3 mutations and often harbor alterations in other pathways, such as TP53 or RB1, which are not amenable to FGFR-targeted therapy. This divergence highlights the importance of tailoring treatment strategies based on the molecular profile of the tumor.

The process of patient selection begins with molecular profiling, which has become an integral component of precision oncology. Techniques such as next-generation sequencing (NGS), polymerase chain reaction (PCR), and fluorescence in situ hybridization (FISH) allow for the detailed characterization of FGFR3 mutations and fusions. These methods are now being integrated into clinical workflows to identify patients most likely to benefit from FGFR inhibitors. Molecular profiling not only provides a clear indication of whether FGFR3-driven signaling is a key oncogenic driver in a given patient's tumor but also helps exclude those without such alterations, thereby avoiding unnecessary treatment and potential adverse effects.

The importance of patient selection is further underscored by the response rates observed in clinical trials of FGFR inhibitors in bladder cancer. For example, erdafitinib, an FGFR inhibitor, has demonstrated significant clinical activity in FGFR3-mutant and FGFR3-fusion-positive bladder cancers. However, its efficacy is largely confined to patients whose tumors harbor these specific genetic changes. In contrast, patients without FGFR alterations derive little to no benefit from FGFR-targeted therapy, underscoring the futility of administering these drugs indiscriminately. This selective efficacy necessitates a precision medicine approach, ensuring that treatment is directed only to those patients with the potential to respond.

Another critical aspect of patient selection is its role in addressing the issue of treatment resistance. Even among patients with FGFR3 alterations, resistance to FGFR inhibitors can develop due to secondary mutations in FGFR3, activation of compensatory signaling pathways, or changes in the tumor microenvironment. While molecular profiling at the time of diagnosis can identify patients with FGFR3-driven tumors, ongoing monitoring may be required to detect emerging resistance mechanisms and adapt treatment strategies accordingly. In the absence of initial patient selection, the likelihood of encountering resistance and suboptimal outcomes would be even greater, further diminishing the therapeutic value of FGFR inhibitors.

Beyond optimizing efficacy, patient selection is also essential for minimizing the risks associated with FGFR-targeted therapies. Like all targeted therapies, FGFR inhibitors are associated with a range of side effects, including hyperphosphatemia, nail and skin changes, eye toxicities, fatigue, and gastrointestinal disturbances. While these side effects are generally manageable, their occurrence in patients unlikely to benefit from treatment represents an unnecessary burden. By ensuring that only patients with FGFR3 alterations are exposed to these drugs, the risk-benefit ratio of FGFR-targeted therapy is significantly improved.

The economic implications of patient selection further emphasize its importance. FGFR inhibitors are costly, and their use in unselected populations could lead to significant healthcare expenditures without commensurate clinical benefit. By focusing treatment on patients with FGFR3 alterations, healthcare systems can allocate resources more efficiently, directing these therapies to those most likely to benefit. This approach not only enhances individual patient outcomes but also supports the sustainability of healthcare systems by reducing the financial burden associated with ineffective treatments.

Moreover, patient selection plays a vital role in clinical research and the development of FGFR inhibitors. Clinical trials of these therapies have increasingly emphasized the need for molecular stratification of participants, ensuring that study populations are enriched for patients with FGFR3-driven tumors. This stratification improves the likelihood of demonstrating the efficacy of FGFR inhibitors, accelerates the regulatory approval process, and provides clearer guidance on their clinical application. Without robust patient selection, clinical trials risk being confounded by the inclusion of FGFR-negative patients, potentially diluting the observed treatment effects and hindering the advancement of these therapies.

Finally, the relevance of patient selection extends to the broader context of personalized medicine. The use of FGFR inhibitors in NMIBC exemplifies the shift away from a one-size-fits-all approach to cancer treatment toward a model in which therapies are tailored to the molecular characteristics of the tumor. This paradigm shift has profound implications for how bladder cancer is managed, offering the potential for improved outcomes through more precise and individualized treatment strategies. By identifying and targeting FGFR3-driven tumors, patient selection serves as a critical enabler of this personalized approach, ensuring that the promise of precision oncology is realized in clinical practice.

In conclusion, patient selection is a fundamental aspect of FGFR-targeted therapy in NMIBC. It ensures that treatment is directed to those most likely to benefit, maximizes therapeutic efficacy, minimizes unnecessary side effects, and supports the cost-effectiveness of these interventions. As molecular profiling technologies become increasingly accessible and integrated into routine care, the ability to identify patients with FGFR3 alterations will continue to improve, further enhancing the potential of FGFR inhibitors to transform the management of NMIBC. By emphasizing the importance of patient selection, the field of oncology is taking a critical step toward delivering more precise, effective, and equitable cancer care.

The present invention aims to assist patient selection to identify those patients that are likely to respond positively to FGFR inhibitor mediated treatment. The inventor has identified genes whose expression levels are predictive for this. In order to do so expression levels of these respective genes were analyzed in 18 different bladder cancer cell lines and correlated to the IC50 values of three different FGFR inhibitors with which these cells were treated. The data is represented in Example 1 and Figs. 1-6, and show a strong correlation between the ImmunoScore gene signature, as well as the single gene expression level PDE4D with the IC50 values determined in the different cell lines. These data strongly suggest that PDE4D expression level or the ImmunoScore gene signature may be used to predict treatment success of a FGFR inhibitor based therapy for an individual subject with bladder cancer.

The invention thus broadly describes a method for predicting effectiveness of a therapy based on FGFR inhibitors for a subject suffering from bladder cancer. This may be done using the PDE4D expression level or the ImmunoScore gene signature. The method provides a prediction if the FGFR inhibitor based therapy will be effective or not. Therefore, in an aspect the invention relates to a method for predicting the effectiveness of FGFR inhibitor based therapy in the treatment of bladder cancer as broadly described herein. The invention further relates to FGFR inhibitors for use in the treatment of bladder cancer when the method as broadly described herein predicts such therapy likely to be effective. Thus the invention relates to stratification of bladder cancer patients in those who are likely to respond to FGFR inhibitor based therapy and those who are less likely to respond to FGFR inhibitor based therapy, as well as FGFR inhibitors for use in the treatment of those who are likely to respond.

Therefore, in a first aspect the invention relates to a Fibroblast Growth Factor Receptor (FGFR) inhibitor for use in the treatment of bladder cancer in a subject in need thereof, the use comprising determining in a bladder cancer sample obtained from the subject the expression level of PDE4D, and administering the FGFR inhibitor to the subject if the expression levels is above a predetermined threshold.

In an aspect the invention relates to a method of treating a subject with bladder cancer, the method comprising determining in a bladder cancer sample obtained from the subject the expression level of PDE4D, and administering the FGFR inhibitor to the subject if the expression levels is above a predetermined threshold.

In a second aspect the invention relates to Fibroblast Growth Factor Receptor (FGFR) inhibitor for use in the treatment of bladder cancer in a subject in need thereof, the use comprising determining in a bladder cancer sample obtained from the subject the expression levels of three or more, for example three, four, five, six, seven, eight, nine, ten, or all eleven, genes selected from APOBEC3A, CIAO1, DDX58, DHX9, IFI16, IFIH1, LRRFIP1, OAS1, ZBP1, EZR, and PDE4D, and calculating a FGFR inhibitor treatment score based on the three or more expression levels, and administering the FGFR inhibitor to the subject if the FGFR inhibitor treatment score is below a predetermined threshold.

In an aspect the invention relates to a method of treating a subject with bladder cancer, the method comprising determining in a bladder cancer sample obtained from the subject the expression levels of three or more, for example three, four, five, six, seven, eight, nine, ten, or all eleven, genes selected from APOBEC3A, CIAO1, DDX58, DHX9, IFI16, IFIH1, LRRFIP1, OAS1, ZBP1, EZR, and PDE4D, and calculating a FGFR inhibitor treatment score based on the three or more expression levels, and administering the FGFR inhibitor to the subject if the FGFR inhibitor treatment score is below a predetermined threshold.

Bladder cancer is any of several types of cancer arising from the tissues of the urinary bladder. Symptoms include blood in the urine, pain with urination, and low back pain. It is caused when epithelial cells that line the bladder become malignant. Risk factors for bladder cancer include smoking, family history, prior pelvic radiation therapy, frequent bladder infections, and exposure to certain chemicals. The most common type is transitional cell carcinoma. Other types include squamous cell carcinoma and adenocarcinoma. Diagnosis is typically by cystoscopy with tissue biopsies. Staging of the cancer is determined by transurethral resection of the bladder tumor (TURBT) and medical imaging.

Non-muscle-invasive bladder cancer (NMIBC) is primarily treated by surgically removing all tumors by TURBT in the same procedure used to collect biopsy tissue for diagnosis. For those with a relatively low risk of tumors recurring, a single dose of chemotherapy (mitomycin C, epirubicin, or gemcitabine) injected into the bladder within 24 hours of TURBT reduces the risk of tumor occurrence by about 39%. Those with higher risk are instead treated with bladder injections of the BCG vaccine (a live bacterial vaccine, traditionally used for tuberculosis), administered weekly for six weeks.

In an embodiment the bladder cancer is non-muscle invasive bladder cancer, muscle invasive bladder cancer or metastatic bladder cancer. Preferably the bladder cancer is NMIBC.

In an embodiment the uses and methods described herein further include determining if the subject has a FGFR mutation. In an embodiment the presence of a FGFR mutation is determined in the bladder cancer sample.

When used herein the term subject refers to a human or a non-human animal such as a mammal. When used herein the subject is a subject suffering from bladder cancer. The subject may be a patient, hence the terms subject and patient are used interchangeably herein. In an embodiment the subject has a FGFR mutation, preferably a FGFR3 mutation.

The presence of FGFR mutations may be used to determine if an FGFR inhibitor based therapy should be considered. Therefore in an embodiment the subject has one or more FGFR mutations. Such mutations can be determined in the bladder cancer sample as described herein. When used herein the term "FGFR" mutation refers to a genetic mutation leading to the substitution, deletion or insertion of one or more amino acids, a frame shift mutation, a gene fusion, or an epigenetic change leading to increased expression of a FGFR gene. In an embodiment the mutation is a substitution or deletion of Lysine 650 of FGFR3 or a substitution or deletion of Glycine 679 of FGFR3 or a substitution or deletion of Asparagine 540 of FGFR3. In an embodiment the mutation is a FGFR3 substitution selected from E466K, A500T, I538F, I538V, N540K, N540S, V555M, P572A, C582F, D617G, E627D, V630M, G637W, D641G, D641N, H643D, D646Y, Y647C, K650E, K650N, N653H, R669G, R669Q, V677I, and G697C, or a corresponding substitution in FGFR1, FGFR2, or FGFR4. What are the corresponding substitutions in FGFR1, FGFR2, or FGFR4 can easily be determined based on sequence homology of the receptors.

The fibroblast growth factor receptors (FGFR) are receptors that bind to members of the fibroblast growth factor (FGF) family of proteins. Some of these receptors are involved in pathological conditions. For example, a point mutation in FGFR3 can lead to achondroplasia. Five distinct membrane FGFR have been identified in vertebrates and all of them belong to the tyrosine kinase superfamily (FGFR1 to FGFR4), these are FGFR1 (Fibroblast growth factor receptor 1, also known as CD331); FGFR2 (Fibroblast growth factor receptor 2, also known as CD332); FGFR3 (Fibroblast growth factor receptor 3, also known as CD333); FGFR4 (Fibroblast growth factor receptor 4, also known as CD334); and FGFRL1 (Fibroblast growth factor receptor-like 1). The FGF/FGFR signaling pathway is involved in a variety of cancers. There are non-selective FGFR inhibitors that act on all of FGFR1-4 and other proteins, and some selective FGFR inhibitors for some/all of FGFR1-4. Selective FGFR inhibitors include AZD4547, BGJ398, JNJ42756493, and PD173074.

Fibroblast Growth Factor Receptor (FGFR) inhibitors (also referred herein as FGFRi) are targeted therapies designed to interfere with the FGFR signaling pathways implicated in various cancers.

Therefore in an embodiment FGFR inhibitor is a compound or biological that impedes the activity of the FGF Receptor. For example the FGFR inhibitor may prevent binding of the FGF receptor to a ligand (e.g. prevent binding of one or more types of FGF to the FGFR), may prevent dimerization of the FGF Receptor, may block tyrosine kinase activity of the FGF receptor or may block downstream signaling, such as but not limited to blocking of docking proteins to phosphorylated sites at the FGF Receptor (e.g. FRS2, PRKCG and GRB2), or blocking activation of such docking proteins.

The FGFR inhibitor may be a non-specific inhibitor which inhibits multiple targets, for example tyrosine kinase receptors, but also inhibits FGFR. Alternatively the FGFR inhibitor may be specific for the FGF Receptor. As a more specific inhibitor generally leads to less side effects when used as a therapy, an FGFR specific inhibitor is preferred. When used herein, an inhibitor is deemed specific for FGFR when the IC50 value of the compound is significantly lower for FGFR compared to other tyrosine kinase receptors. For example the IC50 is a factor 2, 5, 10, 20, 50, 100, 200, 500 or even a factor 1000 lower. A lower IC50 indicates that a lower concentration of the inhibitor results in a 50% reduction of activity, and thus means a stronger effect of the inhibitor on the target. In bladder cancer the most frequently mutated FGF receptor is FGFR3, therefore in an embodiment the FGFR inhibitor is specific for FGFR3. In an embodiment the FGFR inhibitor blocks FGFR3 activity.

In an embodiment the FGFR inhibitor reduces cancer cell proliferation by inhibiting FGFR activity, preferably by FGFR3 activity. When used herein the term "activity" when referring to a FGFR, indicates kinase activity of the FGFR and particularly the ability to initiate downstream signaling. Downstream signaling includes but is not limited to recruitment of docking proteins to the active receptor, activation of such docking proteins, activation and/or translocation to the nucleus of secondary messengers, and activation or inhibition of transcription of FGFR target genes.

Thus in a particularly preferred embodiment the FGFR inhibitor inhibits FGFR activity, preferably FGFR3 activity, and reduces bladder cancer cell proliferation. In an embodiment the bladder cancer cell proliferation is reduced when a bladder cancer cell is contacted with the FGFR inhibitor. In an embodiment the bladder cancer cell is contacted with the FGFR inhibitor in vivo by administration of the FGFR inhibitor to a subject suffering from bladder cancer.

Several FGFR inhibitors have received clinical approval for specific cancer treatments: Erdafitinib (Balversa): Approved by the U.S. Food and Drug Administration (FDA) in April 2019, erdafitinib is indicated for adult patients with locally advanced or metastatic urothelial carcinoma harboring susceptible FGFR3 or FGFR2 genetic alterations. This approval marked the first FGFR kinase inhibitor sanctioned by the FDA. Pemigatinib (Pemazyre): In April 2020, the FDA approved pemigatinib for the treatment of adults with previously treated, unresectable locally advanced or metastatic cholangiocarcinoma (bile duct cancer) characterized by FGFR2 fusions or rearrangements. Futibatinib (Lytgobi): The FDA granted accelerated approval to futibatinib in September 2022 for adults with previously treated, unresectable, locally advanced, or metastatic intrahepatic cholangiocarcinoma harboring FGFR2 gene fusions or other rearrangements. Infigratinib (Truseltiq): Approved by the FDA in May 2021, infigratinib is indicated for adults with previously treated, unresectable locally advanced or metastatic cholangiocarcinoma with FGFR2 fusions or other rearrangements.

Several FGFR inhibitors are currently under development, aiming to expand treatment options for patients with FGFR-driven malignancies. Notable FGFR inhibitors in development include: Resigratinib (KIN-3248): An investigational pan-FGFR inhibitor targeting FGFR1-4, resigratinib is being evaluated in early-phase clinical trials for its efficacy against cancers harboring FGFR alterations. Surufatinib (Sulfatinib): Targeting FGFR1, surufatinib is under investigation for the treatment of various solid tumors, including neuroendocrine tumors. Lucitanib: An inhibitor of FGFR1 and FGFR2, lucitanib has undergone clinical trials for advanced solid tumors. Dovitinib (TKI258): An inhibitor of FGFR1, FGFR2, and FGFR3, dovitinib has had a clinical trial on FGFR-amplified breast cancers. These investigational agents represent the ongoing efforts to develop effective FGFR-targeted therapies for various cancers. Their clinical efficacy and safety profiles are being evaluated in ongoing studies, with the potential to offer new treatment options for patients with FGFR-driven malignancies.

In addition, the following FGFR inhibitors are described in the art: lenvatinib (E7080), ponatinib (AP24534), regorafenib (BAY 73-4506), cediranib (AZD2171), intedanib (BIBF 1120), Fexagratinib (AZD4547), PD173074, FGFR 3831, and brivanib (BMS-540215).

Thus in an embodiment the FGFR inhibitor is selected from erdafitinib, pemigatinib, futibatinib, infigratinib, resigratinib (KIN-3248), surufatinib, lucitanib(E3810), dovitinib(TKI258), lenvatinib (E7080), ponatinib (AP24534), regorafenib (BAY 73-4506), cediranib (AZD2171), intedanib (BIBF 1120), Fexagratinib (AZD4547), PD173074, FGFR 3831, and brivanib (BMS-540215). In a preferred embodiment the FGFR inhibitor is selected from erdafitinib, pemigatinib, futibatinib, infigratinib, resigratinib (KIN-3248), surufatinib, lucitanib(E3810), and dovitinib(TKI258). In a more preferred embodiment the FGFR inhibitor is selected from erdafitinib, pemigatinib, futibatinib, and infigratinib.

In an embodiment the FGFR inhibitor may be provided in addition to other therapy. Typical treatment options for bladder cancer are surgery, radiation therapy chemotherapy, immunotherapy, and target therapy. Thus in one embodiment the use comprises administering the FGFRi inhibitor combined with a therapy selected from surgery, radiation therapy chemotherapy, immunotherapy, and target therapy.

Surgery is the main treatment for bladder cancer. The type of surgery depends on where the cancer is located. Other treatments may be given in addition to surgery. Non limiting examples of surgery include Transurethral resection (TUR) with fulguration, Partial cystectomy, and Radical cystectomy with urinary diversion.

When used herein TUR with fulguration refers to a therapy where a cystoscope is inserted into the bladder through the urethra to remove the cancer or to burn the tumor away with high-energy electricity. When used herein the partial cystectomy refers to surgery to remove part of the bladder. This may be done for patients who have a low-grade tumor that has invaded the wall of the bladder but is limited to one area of the bladder. When used herein Radical Cystectomy refers to surgery to remove the bladder and any lymph nodes and nearby organs that contain cancer. This surgery may be done when the bladder cancer invades the muscle layers or when non-muscle-invasive bladder cancer involves a large part of the bladder.

Chemotherapy, when used herein refers to drugs to stop the growth of cancer cells, either by killing the cells or by stopping them from dividing. Chemotherapy may be combined with further treatment. The chemotherapy may be systemic or intravesical. Systemic chemotherapy for bladder cancer means that chemotherapy drugs are injected into a vein. Non limiting chemotherapies suitable for bladder cancer are carboplatin, cisplatin, doxorubicin, fluorouracil (5-FU), gemcitabine, methotrexate, mitomycin, paclitaxel, and vinblastine. Intravesical chemotherapy means that the drug is administered to the bladder through a tube inserted into the urethra. Intravesical treatments flush the bladder with drugs that kill cancer cells that remain after surgery. In addition, the chemotherapy may be selected from Alkylating agents such as Altretamine, Bendamustine, Busulfan, Carboquone, Carmustine, Chlorambucil, Chlormethine, Chlorozotocin, Cyclophosphamide, Dacarbazine, Fotemustine, Ifosfamide, Lomustine, Melphalan, Melphalan flufenamide, Mitobronitol, Nimustine, Nitrosoureas, Pipobroman, Ranimustine, Semustine, Streptozotocin, Temozolomide, Thiotepa, Treosulfan, Triaziquone, Triethylenemelamine, Trofosfamide, and Uramustine; or Anthracyclines such as Aclarubicin, Daunorubicin, Doxorubicin, Epirubicin, Idarubicin, Mitoxantrone, Pirarubicin, Valrubicin, and Zorubicin; or Cytoskeletal disruptors (taxanes) such as Abraxane, Cabazitaxel, Docetaxel, Larotaxel, Paclitaxel, Taxotere, and Tesetaxel; or Epothilones such as Ixabepilone; or Histone deacetylase inhibitors such as Entinostat, Romidepsin, Vorinostat, and Zabadinostat; or Inhibitors of topoisomerase I such as Belotecan, Camptothecin, Exatecan, Gimatecan, Irinotecan, and Topotecan; or Inhibitors of topoisomerase II such as Etoposide, Teniposide, and Tafluposide; or Kinase inhibitors such as Bortezomib, Erlotinib, Gefitinib, Imatinib, Vemurafenib, and Vismodegib; or Nucleotide analogs and precursor analogs such as Azacitidine, Azathioprine, Capecitabine, Cladribine, Clofarabine, Cytarabine, Decitabine, Doxifluridine, Fludarabine, Fluorouracil, Gemcitabine, Hydroxyurea, Mercaptopurine, Methotrexate, Nelarabine, Pemetrexed, and Tioguanine (formerly Thioguanine); or Peptide antibiotics such as Actinomycin, and Bleomycin; or Platinum-based agents such as Main Carboplatin, Cisplatin, Dicycloplatin, Oxaliplatin, Nedaplatin, and Satraplatin; or Retinoids such as Alitretinoin, Bexarotene, and Tretinoin; or Vinca alkaloids and derivatives such as Vinblastine, Vincristine, Vindesine, and Vinorelbine.

Immunotherapy is a therapy the triggers or supports the immune system to kill cancerous cells. Immunotherapy may be systemic or intravesical. Non limited examples of systemic immunotherapies are atezolizumab, avelumab, nivolumab, and pembrolizumab. Non limiting examples of intravesical immunotherapies are BCG (bacillus Calmette-Guérin), nadofaragene firadenovec-vncg, and nogapendekin alfa inbakicept-pmln, these are typically combined with surgery to kill remaining cancer cells. In addition the immunotherapy may be a cancer vaccine such as but not limited to Sipuleucel-T (Provenge) and prostatic acid phosphatase (PAP), or an Immune checkpoint inhibitors selected from Ipilimumab, Nivolumab, Pembrolizumab, Cemiplimab, Spartalizumab, Camrelizumab, Sintilimab, Tislelizumab, Toripalimab, Dostarlimab, Retifanlimab, AMP-224, MEDI0680, Atezolizumab, Avelumab, Durvalumab, Envafolimab, Cosibelimab, AUNP-12, CA-170, anti-OX40 antibody BMS 986178, Tremelimumab, Abatacept, Belatacept, Sotorasib, Adagrasib, Relatlimab, Lisavanbulin, PHI-101, and Quemliclustat.

Targeted therapy uses drugs or other substances to block the action of specific enzymes, proteins, or other molecules involved in the growth and spread of cancer cells. FGFR inhibitors are a form of targeted therapy, however when referred herein to a combination of an FGFR inhibitor and targeted therapy, the target therapy refers to a therapy targeting a enzymes, proteins, or other molecules which is not FGFR. Non-limiting examples of other targets include the VEGF Receptor. Non-limiting examples are enfortumab vedotin, ramucirumab, and sacituzumab govitecan-hziy. In addition, the targeted therapy may refer to targeted radionuclide therapy. Targeted radionuclide therapy refers to radioisotopes bound to or incorporated in a targeting agent or an antibody such as a nanobody. Targeted therapy may also refer to a therapy that acts on a protein or pathway particularly relied on by cancer cells. For example a targeted therapy may target PARP (PARP inhibitors). Non limiting examples of PARP inhibitors are Rucaparib, Olaparib, Talazoparib, and Niraparib plus abiraterone.

The expression levels are determined in a bladder cancer sample obtained from the subject. When used herein the term bladder cancer sample refers to a sample comprising at least one bladder cancer cell. The sample may for example be a tumor biopsy, part of a resected bladder, a tissue resection, or a blood sample comprising circulating tumor cells. In addition, the bladder cancer sample may include body tissue and/or a fluid, such as, but not limited to, blood (or blood derived such as serum, plasma, or PBMC's (peripheral blood mononuclear cells)), sweat, saliva, urine, bone marrow, and a needle biopsy or resection biopsy, preferably comprising at least one prostate cancer cell. Furthermore, the biological sample may contain a cell extract derived from or a cell population including a cancerous cell or a cell derived from tissue suspected to be cancerous, such as a myeloid cell. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumor cells, extracellular vesicles, a sample containing prostate secreted exosomes, or cell lines or cancer cell line.

In one particular realization, a bladder cancer sample such as a biopsy or resection sample may be obtained and/or used. Such samples may include bladder cancer cells or cell lysates.

It is also conceivable that the content of a bladder cancer sample is submitted to an enrichment step. For instance, a sample may be contacted with ligands specific for the cell membrane or organelles of certain cell types, e.g., tumor cells or myeloid cells, functionalized for example with magnetic particles. The material concentrated by the magnetic particles may subsequently be used for detection and analysis steps as described herein above or below.

Furthermore, bladder cancer cells, e.g., tumor cells, may be enriched via filtration processes of fluid or liquid samples, e.g., blood, urine, etc. Such filtration processes may also be combined with enrichment steps based on ligand specific interactions as described herein above.

When used herein the term bladder cancer cell refers to a tumor cell derived from the bladder or a metastasis thereof. It is preferred that a bladder cancer sample as provided herein is obtained from the subject before the start of the therapy. It is also preferred that said biological sample is a bladder sample or a bladder cancer sample. Thus, in a preferred embodiment the method according to the invention comprises that the bladder cancer sample is obtained from the subject before the start of the therapy, preferably wherein the bladder cancer sample is a bladder sample or a sample comprising bladder cancer cells from the subject. Alternatively, the method according to the invention comprises providing the bladder cancer sample obtained from the subject before the start of the therapy, preferably wherein the bladder cancer sample is a bladder sample or a sample comprising bladder cancer cells from the subject.

In an embodiment the bladder cancer sample is a resected bladder or part thereof, a tumor biopsy, a bladder biopsy, a tissue resection, or any other sample obtained from the patient comprising at least one bladder cancer cell.

When used herein the term "PDE4D" refers to the human cAMP-specific 3',5'-cyclic phosphodiesterase 4D gene (Ensembl: ENSG00000113448), for example, to the sequence as defined in NCBI Reference Sequence NM_001104631.2, specifically, to the nucleotide sequence as set forth in SEQ ID NO:32, which correspond to the sequences of the above indicated NCBI Reference Sequences of the PDE4D transcript, and also relates to the corresponding amino acid sequence for example as set forth in SEQ ID NO:33, which correspond to the protein sequences defined in NCBI Protein Accession Reference Sequence NP_001098101.1 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being at least 75%, 80%, 85%, 90%, 91%, 92%, 93%, 94%, 95%, 96%, 97%, 98% or 99% identical to the sequence as set forth in SEQ ID NO:33 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:32.

When used herein the term PDE4D may also refer to one or more of the specific described PDE4D isoforms, for example one or more of PDE4D1, PDE4D2, PDE4D3, PDE4D4, PDE4D5, PDE4D6, PDE4D7, PDE4D8 and PDE4D9. The when referring to PDE4D expression levels that expression levels may be the cumulative expression levels of two or more, for example two, three, four, five, six, seven, eight or all nine of the expression levels of individual isoforms selected from PDE4D1, PDE4D2, PDE4D3, PDE4D4, PDE4D5, PDE4D6, PDE4D7, PDE4D8 and PDE4D9.

The term "phosphodiesterase 4D1" or "PDE4D1" relates to the splice variant 1 of the human phosphodiesterase PDE4D, i.e. the human phosphodiesterase PDE4D1 gene, preferably to the sequence as defined in NCBI Reference Sequence: NM_001197222.1, more preferably to the nucleotide sequence as set forth in SEQ ID NO: 1, which corresponds to the sequence of the above indicated NCBI Reference Sequence of the PDE4D1 transcript, and also relates to the corresponding amino acid sequence as set forth in SEQ ID NO: 2, which corresponds to the protein sequence defined in NCBI Protein Accession Reference Sequence NP_001184151.1 encoding the PDE4D1 polypeptide. The term "phosphodiesterase 4D1" or "PDE4D1" also relates to the amplicon that can be generated by the primer pair PDE1D1D2_forward (SEQ ID NO: 20) and the PDE1D1D2_reverse (SEQ ID NO: 21) and can be detected by probe SEQ ID NO: 22.

The term "phosphodiesterase 4D2" or "PDE4D2" relates to the splice variant 2 of the human phosphodiesterase PDE4D, i.e. the human phosphodiesterase PDE4D2 gene, preferably to the sequence as defined in NCBI Reference Sequence: NM_001197221.1, more preferably to the nucleotide sequence as set forth in SEQ ID NO: 3, which corresponds to the sequence of the above indicated NCBI Reference Sequence of the PDE4D2 transcript, and also relates to the corresponding amino acid sequence as set forth in SEQ ID NO: 4, which corresponds to the protein sequence defined in NCBI Protein Accession Reference Sequence NP _001184150.1 encoding the PDE4D2 polypeptide. The term "phosphodiesterase 4D2" or "PDE4D2" also relates to the amplicon that can be generated by the primer pair PDE1D1D2_forward (SEQ ID NO: 20) and the PDE1D1D2_reverse (SEQ ID NO: 21) and can be detected by probe SEQ ID NO: 22.

The term "phosphodiesterase 4D3" or "PDE4D3" relates to the splice variant 3 of the human phosphodiesterase PDE4D, i.e. the human phosphodiesterase PDE4D3 gene, preferably to the sequence as defined in NCBI Reference Sequence: NM_006203.4, more preferably to the nucleotide sequence as set forth in SEQ ID NO: 5, which corresponds to the sequence of the above indicated NCBI Reference Sequence of the PDE4D3 transcript, and also relates to the corresponding amino acid sequence as set forth in SEQ ID NO: 6, which corresponds to the protein sequence defined in NCBI Protein Accession Reference Sequence NP_006194.2 encoding the PDE4D3 polypeptide.

The term "phosphodiesterase 4D4" or "PDE4D4" relates to the splice variant 4 of the human phosphodiesterase PDE4D, i.e. the human phosphodiesterase PDE4D4 gene, preferably to the sequence as defined in NCBI Reference Sequence: NM_001104631.1, more preferably to the nucleotide sequence as set forth in SEQ ID NO: 7, which corresponds to the sequence of the above indicated NCBI Reference Sequence of the PDE4D4 transcript, and also relates to the corresponding amino acid sequence as set forth in SEQ ID NO: 8, which corresponds to the protein sequence defined in NCBI Protein Accession Reference Sequence NP_001098101.1 encoding the PDE4D4 polypeptide.

The term "phosphodiesterase 4D5" or "PDE4D5" relates to the splice variant 5 of the human phosphodiesterase PDE4D, i.e. the human phosphodiesterase PDE4D5 gene, preferably to the sequence as defined in NCBI Reference Sequence: NM_001197218.1, more preferably to the nucleotide sequence as set forth in SEQ ID NO: 9, which corresponds to the sequence of the above indicated NCBI Reference Sequence of the PDE4D5 transcript, and also relates to the corresponding amino acid sequence as set forth in SEQ ID NO: 10, which corresponds to the protein sequence defined in NCBI Protein Accession Reference Sequence NP _001184147.1 encoding the PDE4D5 polypeptide. The term "phosphodiesterase 4D5" or "PDE4D5" also relates to the amplicon that can be generated by the primer pair PDE1D5_forward (SEQ ID NO: 23) and the PDE1D5_reverse (SEQ ID NO: 24) and can be detected by probe SEQ ID NO: 25.

The term "phosphodiesterase 4D6" or "PDE4D6" relates to the splice variant 6 of the human phosphodiesterase PDE4D, i.e. the human phosphodiesterase PDE4D6 gene, preferably to the sequence as defined in NCBI Reference Sequence: NM_001197223.1, more preferably to the nucleotide sequence as set forth in SEQ ID NO: 11, which corresponds to the sequence of the above indicated NCBI Reference Sequence of the PDE4D6 transcript, and also relates to the corresponding amino acid sequence as set forth in SEQ ID NO: 12, which corresponds to the protein sequence defined in NCBI Protein Accession Reference Sequence NP _001184152.1 encoding the PDE4D6 polypeptide.

The term "phosphodiesterase 4D7" or "PDE4D7" relates to the splice variant 7 of the human phosphodiesterase PDE4D, i.e. the human phosphodiesterase PDE4D7 gene, preferably to the sequence as defined in NCBI Reference Sequence: NM_001165899.1, more preferably to the nucleotide sequence as set forth in SEQ ID NO: 13, which corresponds to the sequence of the above indicated NCBI Reference Sequence of the PDE4D7 transcript, and also relates to the corresponding amino acid sequence as set forth in SEQ ID NO: 14, which corresponds to the protein sequence defined in NCBI Protein Accession Reference Sequence NP_001159371.1 encoding the PDE4D7 polypeptide. The term "phosphodiesterase 4D7" or "PDE4D7" also relates to the amplicon that can be generated by the primer pair PDE1D7_forward (SEQ ID NO: 26) and the PDE1D7_reverse (SEQ ID NO: 27) and can be detected by probe SEQ ID NO: 28.

The term "phosphodiesterase 4D8" or "PDE4D8" relates to the splice variant 8 of the human phosphodiesterase PDE4D, i.e. the human phosphodiesterase PDE4D8 gene, preferably to the sequence as defined in NCBI Reference Sequence: NM_001197219.1, more preferably to the nucleotide sequence as set forth in SEQ ID NO: 15, which corresponds to the sequence of the above indicated NCBI Reference Sequence of the PDE4D8 transcript, and also relates to the corresponding amino acid sequence as set forth in SEQ ID NO: 16, which corresponds to the protein sequence defined in NCBI Protein Accession Reference Sequence NP _001184148.1 encoding the PDE4D8 polypeptide.

The term "phosphodiesterase 4D9" or "PDE4D9" relates to the splice variant 9 of the human phosphodiesterase PDE4D, i.e. the human phosphodiesterase PDE4D9 gene, preferably to the sequence as defined in NCBI Reference Sequence: NM_001197220.1, more preferably to the nucleotide sequence as set forth in SEQ ID NO: 17, which corresponds to the sequence of the above indicated NCBI Reference Sequence of the PDE4D9 transcript, and also relates to the corresponding amino acid sequence as set forth in SEQ ID NO: 18, which corresponds to the protein sequence defined in NCBI Protein Accession Reference Sequence NP _001184149.1 encoding the PDE4D9 polypeptide. The term "phosphodiesterase 4D9" or "PDE4D9" also relates to the amplicon that can be generated by the primer pair PDE1D5_forward (SEQ ID NO: 29) and the PDE1D5_reverse (SEQ ID NO: 30) and can be detected by probe SEQ ID NO: 31.

The terms "PDE4D1", "PDE4D2", "PDE4D3", "PDE4D4", "PDE4D5", "PDE4D6", "PDE4D7", "PDE4D8" and "PDE4D9" also comprises nucleotide sequences showing a high degree of homology to PDE4D1, PDE4D2, PDE4D3, PDE4D4, PDE4D5, PDE4D6, PDE4D7, PDE4D8 and PDE4D9 respectively, e.g. nucleic acid sequences being at least 75%, 80%, 85%, 90%, 91%, 92%, 93%, 94%, 95%, 96%, 97%, 98% or 99% identical to the sequence as set forth in SEQ ID NOs: 1, 3, 5, 7, 9, 11, 13, 15 or 17 respectively or amino acid sequences being at least 75%, 80%, 85%, 90%, 91%, 92%, 93%, 94%, 95%, 96%, 97%, 98% or 99% identical to the sequence as set forth in SEQ ID NO: 2, 4, 6, 8, 10, 12, 14, 16 or 18 respectively or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 2, 4, 6, 8, 10, 12, 14, 16 or 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1, 3, 5, 7, 9, 11, 13, 15 or 17.

The term "expression level" as used herein refers to the amount of PDE4D variant transcript and/or PDE4D protein derivable from a defined number of cells or a defined tissue portion, preferably to the amount of PDE4D variant transcript and/or PDE4D variant protein obtainable in a standard nucleic acid (e.g. RNA) or protein extraction procedure. Suitable extraction methods are known to the person skilled in the art.

When used herein the term "APOBEC3A" refers to the human Apolipoprotein B mRNA editing enzyme, catalytic polypeptide-like 3A gene (Ensembl: ENSG00000128383), for example, to the sequence as defined in NCBI Reference Sequence NM_145699.4, specifically, to the nucleotide sequence as set forth in SEQ ID NO:34, which correspond to the sequences of the above indicated NCBI Reference Sequences of the APOBEC3A transcript, and also relates to the corresponding amino acid sequence for example as set forth in SEQ ID NO:35, which correspond to the protein sequences defined in NCBI Protein Accession Reference Sequence NP_663745.1 encoding the APOBEC3A polypeptide.

The term "APOBEC3A" also comprises nucleotide sequences showing a high degree of homology to APOBEC3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being at least 75%, 80%, 85%, 90%, 91%, 92%, 93%, 94%, 95%, 96%, 97%, 98% or 99% identical to the sequence as set forth in SEQ ID NO:35 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:34.

When used herein the term "CIAO1" refers to the human Probable cytosolic iron-sulfur protein assembly protein gene (Ensembl: ENSG00000144021), for example, to the sequence as defined in NCBI Reference Sequence NM_004804.3, specifically, to the nucleotide sequence as set forth in SEQ ID NO:36, which correspond to the sequences of the above indicated NCBI Reference Sequences of the CIAO1 transcript, and also relates to the corresponding amino acid sequence for example as set forth in SEQ ID NO:37, which correspond to the protein sequences defined in NCBI Protein Accession Reference Sequence NP_004795.1 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being at least 75%, 80%, 85%, 90%, 91%, 92%, 93%, 94%, 95%, 96%, 97%, 98% or 99% identical to the sequence as set forth in SEQ ID NO:37 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:37 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:36.

When used herein the term "DDX58" refers to the human retinoic acid-inducible gene I (Ensembl: ENSG00000107201), for example, to the sequence as defined in NCBI Reference Sequence NM_014314.4, specifically, to the nucleotide sequence as set forth in SEQ ID NO:38, which correspond to the sequences of the above indicated NCBI Reference Sequences of the DDX58 transcript, and also relates to the corresponding amino acid sequence for example as set forth in SEQ ID NO:39, which correspond to the protein sequences defined in NCBI Protein Accession Reference Sequence NP_055129.2 encoding the DDX58 polypeptide.

The term "DDX58" also comprises nucleotide sequences showing a high degree of homology to DDX58, e.g., nucleic acid sequences being at least 75%, 80%, 85%, 90%, 91%, 92%, 93%, 94%, 95%, 96%, 97%, 98% or 99% identical to the sequence as set forth in SEQ ID NO:38 or amino acid sequences being at least 75%, 80%, 85%, 90%, 91%, 92%, 93%, 94%, 95%, 96%, 97%, 98% or 99% identical to the sequence as set forth in SEQ ID NO:39 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:39 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:38.

When used herein the term "DHX9" refers to the human ATP-dependent RNA helicase A gene (Ensembl: ENSG00000135829), for example, to the sequence as defined in NCBI Reference Sequence NM_001357.5, specifically, to the nucleotide sequence as set forth in SEQ ID NO:40, which correspond to the sequences of the above indicated NCBI Reference Sequences of the DHX9 transcript, and also relates to the corresponding amino acid sequence for example as set forth in SEQ ID NO:41, which correspond to the protein sequences defined in NCBI Protein Accession Reference Sequence NP_001348.2 encoding the DHX9 polypeptide.

The term "DHX9" also comprises nucleotide sequences showing a high degree of homology to DHX9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:40 or amino acid sequences being at least 75%, 80%, 85%, 90%, 91%, 92%, 93%, 94%, 95%, 96%, 97%, 98% or 99% identical to the sequence as set forth in SEQ ID NO:41 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:41 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:40.

When used herein the term "IFI16" refers to the human Gamma-interferon-inducible protein Ifi-16 gene (Ensembl: ENSG00000163565), for example, to the sequence as defined in NCBI Reference Sequence NM_001206567.2, specifically, to the nucleotide sequence as set forth in SEQ ID NO:42, which correspond to the sequences of the above indicated NCBI Reference Sequences of the IFI16 transcript, and also relates to the corresponding amino acid sequence for example as set forth in SEQ ID NO:43, which correspond to the protein sequences defined in NCBI Protein Accession Reference Sequence NP_001193496.1 encoding the IFI16 polypeptide.

The term "IFI16" also comprises nucleotide sequences showing a high degree of homology to IFI16, e.g., nucleic acid sequences being at least 75%, 80%, 85%, 90%, 91%, 92%, 93%, 94%, 95%, 96%, 97%, 98% or 99% identical to the sequence as set forth in SEQ ID NO:42 or amino acid sequences being at least 75%, 80%, 85%, 90%, 91%, 92%, 93%, 94%, 95%, 96%, 97%, 98% or 99% identical to the sequence as set forth in SEQ ID NO:43 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:42.

When used herein the term "IFIH1" refers to the human melanoma differentiation-associated protein 5 gene (Ensembl: ENSG00000115267), for example, to the sequence as defined in NCBI Reference Sequence NM_022168.4, specifically, to the nucleotide sequence as set forth in SEQ ID NO:44, which correspond to the sequences of the above indicated NCBI Reference Sequences of the IFIH1 transcript, and also relates to the corresponding amino acid sequence for example as set forth in SEQ ID NO:45, which correspond to the protein sequences defined in NCBI Protein Accession Reference Sequence NP_071451.2 encoding the IFIH1 polypeptide.

The term "IFIH1" also comprises nucleotide sequences showing a high degree of homology to IFIH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being at least 75%, 80%, 85%, 90%, 91%, 92%, 93%, 94%, 95%, 96%, 97%, 98% or 99% identical to the sequence as set forth in SEQ ID NO:45 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:44.

When used herein the term "LRRFIP1" refers to the human Leucine-rich repeat flightless-interacting protein 1 gene (Ensembl: ENSG00000124831), for example, to the sequence as defined in NCBI Reference Sequence NM_001137550.2, specifically, to the nucleotide sequence as set forth in SEQ ID NO:46, which correspond to the sequences of the above indicated NCBI Reference Sequences of the LRRFIP1 transcript, and also relates to the corresponding amino acid sequence for example as set forth in SEQ ID NO:47, which correspond to the protein sequences defined in NCBI Protein Accession Reference Sequence NP_001131022.1 encoding the LRRFIP1 polypeptide.

The term "LRRFIP1" also comprises nucleotide sequences showing a high degree of homology to LRRFIP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being at least 75%, 80%, 85%, 90%, 91%, 92%, 93%, 94%, 95%, 96%, 97%, 98% or 99% identical to the sequence as set forth in SEQ ID NO:47 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:47 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:46.

When used herein the term "OAS1" refers to the human 2'-5'-oligoadenylate synthetase 1 gene (Ensembl: ENSG00000089127), for example, to the sequence as defined in NCBI Reference Sequence NM_001032409.3, specifically, to the nucleotide sequence as set forth in SEQ ID NO:48, which correspond to the sequences of the above indicated NCBI Reference Sequences of the OAS1 transcript, and also relates to the corresponding amino acid sequence for example as set forth in SEQ ID NO:49, which correspond to the protein sequences defined in NCBI Protein Accession Reference Sequence NP_001027581.1 encoding the OAS1 polypeptide.

The term "OAS1" also comprises nucleotide sequences showing a high degree of homology to OAS1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:48.

When used herein the term "ZBP1" refers to the human Z-DNA-binding protein 1 gene (Ensembl: ENSG00000124256), for example, to the sequence as defined in NCBI Reference Sequence NM_001160417.2, specifically, to the nucleotide sequence as set forth in SEQ ID NO:50, which correspond to the sequences of the above indicated NCBI Reference Sequences of the ZBP1 transcript, and also relates to the corresponding amino acid sequence for example as set forth in SEQ ID NO:51, which correspond to the protein sequences defined in NCBI Protein Accession Reference Sequence NP_001153889.1 encoding the ZBP1 polypeptide.

The term "ZBP1" also comprises nucleotide sequences showing a high degree of homology to ZBP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:50 or amino acid sequences being at least 75%, 80%, 85%, 90%, 91%, 92%, 93%, 94%, 95%, 96%, 97%, 98% or 99% identical to the sequence as set forth in SEQ ID NO:51 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:50.

When used herein the term "EZR" refers to the human Ezrin gene (Ensembl: ENSG00000092820), for example, to the sequence as defined in NCBI Reference Sequence NM_001111077.2, specifically, to the nucleotide sequence as set forth in SEQ ID NO:52, which correspond to the sequences of the above indicated NCBI Reference Sequences of the EZR transcript, and also relates to the corresponding amino acid sequence for example as set forth in SEQ ID NO:53, which correspond to the protein sequences defined in NCBI Protein Accession Reference Sequence NP_001104547.1 encoding the EZR polypeptide.

The term "EZR" also comprises nucleotide sequences showing a high degree of homology to EZR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being at least 75%, 80%, 85%, 90%, 91%, 92%, 93%, 94%, 95%, 96%, 97%, 98% or 99% identical to the sequence as set forth in SEQ ID NO:53 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:53 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:52.

Accordingly, the treatment is conditional on the expression level of PDE4D exceeding a certain threshold or the FGFR inhibitor treatment score being below a certain threshold. The FGFR inhibitor treatment score is based on the expression levels of three or more, for example three, four, five, six, seven, eight, nine, ten or all eleven, genes selected from APOBEC3A, CIAO1, DDX58, DHX9, IFI16, IFIH1, LRRFIP1, OAS1, ZBP1, EZR, and PDE4D. Therefore it is further anticipated that the treatment is conditional on the expression level of PDE4D and one or more gene expression levels selected from three or more genes selected from APOBEC3A, CIAO1, DDX58, DHX9, IFI16, IFIH1, LRRFIP1, OAS1, ZBP1, and EZR. A simple linear model may be used to combine these expression levels to arrive at a FGFR inhibitor treatment score. For example, in a non-limiting embodiment the FGFR inhibitor treatment score is calculated using the formula:

$$FGFRi\_Score = a * [gene\_A] + b * [gene\_B] + c * [gene\_C] + d$$

wherein a, b, c, and d are constants, and [gene_A], [gen_B], and [gene_C] are each a numerical values representing the expression levels of the three or more selected genes, and FGFRi_Score is the FGFR inhibitor treatment score. In case more than 3 gene expression levels are used the additional gene exression levels are multiplies with a constant and added in the equation, for example

$$FGFRi\_Score = a * [gene\_A] + b * [gene\_B] + c * [gene\_C] + d * [gene\_D] + e$$

It is understood that gene_A, gene_B, ... are numerical values representing the expression level of respectively gene_A, gene_B, ... etc. where these genes are selected from APOBEC3A, CIAO1, DDX58, DHX9, IFI16, IFIH1, LRRFIP1, OAS1, ZBP1, EZR, and PDE4D such that none of the genes are the same.

The constants may be "1" or "-1" to create a very simple model wherein the constant reflect a positive or negative correlation with the FGFR inhibitor treatment score. In an embodiment the FGFR inhibitor treatment score is calculated using a linear regression model.

It is further preferred that the determining of expression levels comprises normalizing the gene expression levels with respect to one or more reference genes selected from the group consisting of: hypoxanthine phosphoribosyltransferase 1 (HPRT1), Tubulin-Alpha-1b (TUBA1B), pumilio RNA-Binding Family Member (PUM1), and TATA box binding protein (TBP), wherein the expression levels are determined based on the normalized gene expression profile.

Other reference genes which may be additionally or alternatively used for normalizing the PDE4D gene expression profile include: actin, beta, mRNA (ACTB); 60S acidic ribosomal phosphoprotein P0 mRNA (RPLP0); Polymerase (RNA) II (DNA Directed) Polypeptide A, 220kDa (POLR2A); Beta-2-Microglobulin (B2M); and Aminolevulinate-Delta-Synthase (ALAS-1).

By normalizing the gene expression profile with respect to one or more reference genes and by determining the expression based score is determined based on the normalized gene expression profile, variability in the determination of the expression based risk score can be reduced. This enables differentiation between real variations in gene expression profiles and variations due to the measurement processes. In this respect, it has been found that HPRT1, TUBA1B, PUM1, and TBP are particularly well suited as reference genes for normalizing the expression levels of the genes or isoforms disclosed herein.

Determining the expression levels of the genes or isoforms disclosed may be performed using methods known in the art. For example, the gene expression levels may be determined by detecting mRNA expression using one or more primers and/or probes and/or one or more sets thereof. Moreover, the gene expression profile may be determined by an amplification based method and/or microarray analysis and/or RNA sequencing. The determining of the gene expression profile may include performing Real-Time Quantitative Polymerase Chain Reaction (RT-qPCR) on RNA extracted from the biological sample. In other embodiments, the gene expression profile is determined by RNA sequencing, conventional PCR (using, e.g., end point analysis by gel electrophoresis), or multiplex-PCR. In the case of RT-qPCR, the determining of the gene expression profile may include determining a threshold cycle (Ct) value for genes or isoforms disclosed herein and optionally each of the one or more reference genes. The PCR may be performed with at least one primer and/or probe for measuring a reference gene selected from HPRT1, TUBA1B, PUM1, and TBP.

Preferably the expression levels, e.g. the PDE4D expression level or three or more expression levels selected from APOBEC3A, CIAO1, DDX58, DHX9, IFI16, IFIH1, LRRFIP1, OAS1, ZBP1, EZR, and PDE4D have been or are determined for the bladder cancer patient at the time of taking a biopsy and/or at the time of bladder surgery, such as partial or total removal of the bladder or bladder tumor. Thus ideally the expression levels are determined in a sample obtained during biopsy or bladder or tumor resection. Thus the sample may be taken from the biopsy or resected bladder or tumor, but may also be a separate sample as described herein.

Gene expression levels may be determined using techniques common in the art such a but not limited to RNA sequencing, targeted sequencing, PCR, digital PCR, and quantitative PCR. Gene expression levels may be determined using means for determining expression levels of the respective genes. The means for determining the expression levels of the genes described herein may be primers and/or probes suitable for any one of PCR, quantitative PCR, digital PCR, RNA sequencing, or targeted RNA sequencing. Thus for example the means may be PCR primers, quantitative PCR primers and probes, digital PCR primers and probes, or capture probes for targeted mRNA sequencing. Preferably the means are PCR or qPCR amplification primers and optionally probes or mRNA targeted sequencing capture probes.

The methods described herein use the expression level of PDE4D or three or more genes selected from APOBEC3A, CIAO1, DDX58, DHX9, IFI16, IFIH1, LRRFIP1, OAS1, ZBP1, EZR, and PDE4D. For each of these expression levels it may be or for the FGFR inhibitor treatment score it is determined whether the expression level or the score is above or below a threshold. For example for PDE4D the threshold may be defined as a threshold above which the expression level is considered high and below which the expression level is considered low. The same may be done for each of the three or more expression levels selected from APOBEC3A, CIAO1, DDX58, DHX9, IFI16, IFIH1, LRRFIP1, OAS1, ZBP1, EZR, and PDE4D, and wherein a qualification of the expression levels ("high" or "low") are used as input in the model to calculate the score. Thresholds as described herein can easily be determined in a reference sample, or preferably a cohort of reference samples. For this the expression level is preferably quantified. Quantification may for example be done by normalizing to a household gene or by calculating reads per million, but other suitable method for quantifying gene expression levels are known in the art and may also be suitably used in the method described herein. Thus when used herein the terms "high expression level" or "the expression level is high" can be interpreted as exceeding a threshold expression level for said gene. In this respect it is appreciated that a high expression level will be different for each gene and thus for each gene individually it needs to be determined what constitutes a high expression level, for example by establishing the appropriate threshold above which it is deemed high. The threshold may be determined using methods common in the art. For example from a pool of bladder cancer samples which includes poor and good responders to FGFR inhibitors, expression levels may be collected for the respective gene and the threshold may be set as the mean or average expression level of this pool. Alternatively average expression levels may be determined for responders and non-responder and the threshold may be set as a value in between those average expression level values. The threshold is preferably chosen such as to optimize separation between the good responder and poor responder groups.

In an embodiment the threshold is predetermined in a cohort of bladder cancer samples by correlating the PDE4D expression level or the FGFR inhibitor treatment score to the IC50 of the FGFR inhibitor.

In a third aspect the invention relates to a computer implemented method of predicting effectiveness of treating a subject with bladder cancer with an FGFR inhibitor, the method comprising receiving the expression level of PDE4D determined in a bladder cancer sample obtained from the subject, and predicting the effectiveness of treating the subject with a FGFR inhibitor based on the PDE4D expression level. In an embodiment the invention includes an active step of determining the expression levels and thus relates to a method of predicting effectiveness of treating a subject with bladder cancer with an FGFR inhibitor, the method comprising determining the expression level of PDE4D in a bladder cancer sample obtained from the subject, and predicting the effectiveness of treating the subject with a FGFR inhibitor based on the PDE4D expression level.

In a fourth aspect the invention relates to a computer implemented method of predicting effectiveness of treating a subject with bladder cancer with an FGFR inhibitor, the method comprising receiving the expression levels of three or more, for example three, four, five, six, seven, eight, nine, ten, or all eleven, genes selected from APOBEC3A, CIAO1, DDX58, DHX9, IFI16, IFIH1, LRRFIP1, OAS1, ZBP1, EZR, and PDE4D determined in a bladder cancer sample obtained from the subject, and calculating a FGFR inhibitor treatment score based on the three or more expression levels, and predicting he effectiveness of treating the subject with a FGFR inhibitor based on the FGFR inhibitor treatment score. In an embodiment the invention includes an active step of determining the expression levels and thus relates to a method of predicting effectiveness of treating a subject with bladder cancer with an FGFR inhibitor, the method comprising determining the expression levels of three or more genes selected from APOBEC3A, CIAO1, DDX58, DHX9, IFI16, IFIH1, LRRFIP1, OAS1, ZBP1, EZR, and PDE4D in a bladder cancer sample obtained from the subject, and calculating a FGFR inhibitor treatment score based on the three or more expression levels, and predicting he effectiveness of treating the subject with a FGFR inhibitor based on the FGFR inhibitor treatment score.

The method broadly described herein determines the effectiveness of treating the subject with a FGFR inhibitor based on either PDE4D or the FGFR inhibitor treatment score. When used herein "effectiveness" when referring to treating the subject with a FGFR inhibitor indicates a chance the FGFR inhibitor will make an impact on the bladder cancer. For example the FGFR inhibitor may be considered effective when the inhibitor is predicted to reduce tumor proliferation, stop tumor growth, cause tumor cell death or results in a clinically desirable outcome. For example effectiveness can be based on determined or predicted IC50 values. In an embodiment the determined or predicted LN IC50 (natural logarithm of the IC50 value) is 2.5 or less, preferably 2.0, 1.5, 1.0, 0.5, or even 0.0 or less.

In an embodiment the bladder cancer sample is a sample as described herein above. In an embodiment the bladder cancer sample is a sample comprising at least one bladder cancer cell, preferably wherein the sample is biopsy, or tissue resection of the bladder or a metastasis thereof, or a liquid biopsy.

In an embodiment the FGFR inhibitor treatment score is calculated using the formula:

$$FGFRi\_Score = a * [gene\_A] + b * [gene\_B] + c * [gene\_C] + d$$

wherein a, b, c, and d are constants, and [gene_A], [gen_B], and [gene_C] are each a numerical values representing the expression levels of the three or more selected genes, and FGFRi_Score is the FGFR inhibitor treatment score.

In an embodiment the FGFR inhibitor is selected from erdafitinib, pemigatinib, futibatinib, infigratinib, resigratinib (KIN-3248), surufatinib, lucitanib(E3810), dovitinib(TKI258), lenvatinib (E7080), ponatinib (AP24534), regorafenib (BAY 73-4506), cediranib (AZD2171), intedanib (BIBF 1120), Fexagratinib (AZD4547), PD173074, FGFR 3831, and brivanib (BMS-540215).

In an embodiment the threshold is predetermined in a cohort of bladder cancer samples by correlating the PDE4D expression level or the FGFR inhibitor treatment score to the IC50 of the FGFR inhibitor.

In an embodiment the method is used to provide a treatment recommendation to the subject or a caregiver. In an embodiment the recommended treatment consists or comprises treatment with an FGFR inhibitor when a good effectiveness of the inhibitor is predicted. In an embodiment the treatment is a combination of FGFR inhibitor treatment with one or more of surgery, chemotherapy, radiation therapy, targeted therapy or immunotherapy as broadly described herein above.

In an embodiment the recommended treatment does not include a FGFR inhibitor when a poor effectiveness of the inhibitor is predicted. In an embodiment the treatment is an alternative to FGFR inhibitor treatment. In an embodiment the alternative comprises or consists of one or more of surgery, chemotherapy, radiation therapy, targeted therapy or immunotherapy as broadly described herein above.

In a fifth aspect the invention relates to a kit of parts comprising a FGFR inhibitor and means for determining the expression levels of PDE4D and/or three or more, for example three, four, five, six, seven, eight, nine, ten, or all eleven, genes selected from APOBEC3A, CIAO1, DDX58, DHX9, IFI16, IFIH1, LRRFIP1, OAS1, ZBP1, EZR, and PDE4D in a sample.

In an embodiment the kit is a kit selected from an RNA sequencing kit, a PCR kit, a qPCR kit, a digital PCR kit, an immunohistochemistry kit, an ELISA kit, and an in situ RNA hybridization kit. In an embodiment the kit is for selectively amplifying PDE4D and/or three or more genes selected from APOBEC3A, CIAO1, DDX58, DHX9, IFI16, IFIH1, LRRFIP1, OAS1, ZBP1, EZR, and PDE4D mRNA in a sample. In an embodiment the kit is for detecting PDE4D and/or three or more genes selected from APOBEC3A, CIAO1, DDX58, DHX9, IFI16, IFIH1, LRRFIP1, OAS1, ZBP1, EZR, and PDE4D protein in a sample. In an embodiment the kit is for quantifying PDE4D and/or three or more genes selected from APOBEC3A, CIAO1, DDX58, DHX9, IFI16, IFIH1, LRRFIP1, OAS1, ZBP1, EZR, and PDE4D mRNA or protein. In an embodiment the kit is suitable for use in a method for determining a treatment response to FGFR inhibitors in bladder cancer.

The kit may further comprise buffers, components or enzymes suitable for the envisioned technique, e.g. RNA sequencing, PCR, qPCR, digital PCR, immunohistochemistry, ELISA, or in situ RNA hybridization. Thus the kit may comprise one or more of a polymerase, a buffer suitable for PCR reactions, dNTP stock solutions, a buffer suitable for RNA sequencing, a primary and/or second antibody for ELISA, a reagent or colorant for detecting ELISA signal, and in situ hybridization buffer.

Gene expression levels may be determined using techniques common in the art such a but not limited to RNA sequencing, targeted sequencing, PCR, digital PCR, and quantitative PCR. Gene expression levels may be determined using means for determining expression levels of the respective genes. The means for determining the expression levels of the genes described herein may be primers and/or probes suitable for any one of PCR, quantitative PCR, digital PCR, RNA sequencing, or targeted RNA sequencing. Thus for example the means may be PCR primers, quantitative PCR primers and probes, digital PCR primers and probes, or capture probes for targeted mRNA sequencing. Preferably the means are PCR or qPCR amplification primers and optionally probes or mRNA targeted sequencing capture probes.

In a sixth aspect the invention relates to use of a kit to predict effectiveness of treating a subject with bladder cancer with an FGFR inhibitor, the kit comprising means for determining the expression levels of PDE4D and/or three or more, for example three, four, five, six, seven, eight, nine, ten, or all eleven, genes selected from APOBEC3A, CIAO1, DDX58, DHX9, IFI16, IFIH1, LRRFIP1, OAS1, ZBP1, EZR, and PDE4D in a sample, wherein the use comprises determining the expression levels of PDE4D and/or three or more, for example three, four, five, six, seven, eight, nine, ten, or all eleven, genes selected from APOBEC3A, CIAO1, DDX58, DHX9, IFI16, IFIH1, LRRFIP1, OAS1, ZBP1, EZR, and PDE4D in a bladder cancer sample from a subject with bladder cancer, and predicting the effectiveness of treating the subject with bladder cancer with an FGFR inhibitor based on the PDE4D expression level and/or the expression levels of the three or more, for example three, four, five, six, seven, eight, nine, ten, or all eleven, genes selected from APOBEC3A, CIAO1, DDX58, DHX9, IFI16, IFIH1, LRRFIP1, OAS1, ZBP1, EZR, and PDE4D.

In an embodiment the use involves performing the method of the first or second aspect of the invention as broadly described herein. Thus in an embodiment the invention relates to use of a kit to predict effectiveness of treating a subject with bladder cancer with an FGFR inhibitor, the kit comprising means for determining the expression levels of PDE4D and/or three or more genes selected from APOBEC3A, CIAO1, DDX58, DHX9, IFI16, IFIH1, LRRFIP1, OAS1, ZBP1, EZR, and PDE4D in a sample, wherein the use comprises determining the expression levels of PDE4D and/or three or more genes selected from APOBEC3A, CIAO1, DDX58, DHX9, IFI16, IFIH1, LRRFIP1, OAS1, ZBP1, EZR, and PDE4D in a bladder cancer sample from a subject with bladder cancer, and predicting the effectiveness of treating the subject with bladder cancer with an FGFR inhibitor based on the PDE4D expression level and/or calculating a FGFR inhibitor treatment score based on the three or more expression levels selected from APOBEC3A, CIAO1, DDX58, DHX9, IFI16, IFIH1, LRRFIP1, OAS1, ZBP1, EZR, and PDE4D, and predicting he effectiveness of treating the subject with a FGFR inhibitor based on the FGFR inhibitor treatment score.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

Examples

Having now generally described the invention, the same will be more readily understood through reference to the following examples which is provided by way of illustration and is not intended to be limiting of the present invention.

**Example 1**

*The GDSC1 Dataset*

The Genomics of Drug Sensitivity in Cancer (GDSC) database is a prominent resource that links cancer genomics with therapeutic response, aiming to facilitate precision oncology. The GDSC1 dataset is a foundational dataset in this initiative, providing comprehensive genomic and drug response data from a large panel of human cancer cell lines. It is widely used to identify biomarkers of drug sensitivity and resistance and to uncover potential therapeutic targets.

*Generation of the GDSC1 Dataset*

The GDSC1 dataset was generated through systematic screening of hundreds of cancer cell lines, encompassing a broad spectrum of cancer types. These cell lines were cultured under controlled conditions and exposed to a library of anticancer compounds at varying concentrations. Drug sensitivity was assessed using high-throughput viability assays, which measured the growth inhibition of cell lines upon drug treatment. The resulting dose-response curves were analyzed to compute metrics such as the half-maximal inhibitory concentration (IC50) and area under the curve (AUC), which quantify the drug's efficacy against specific cell lines.

In parallel, comprehensive genomic profiling of the cell lines was conducted. This included the characterization of mutations, copy number variations (CNVs), methylation patterns, and gene expression profiles. Genomic and transcriptomic data were integrated with drug sensitivity results to build predictive models and identify genomic features that correlate with therapeutic responses.

*Purpose of the GDSC1 Dataset*

The GDSC1 dataset was created to bridge the gap between cancer genomics and pharmacology. Its primary purpose is to enable researchers to identify genetic and molecular biomarkers associated with sensitivity or resistance to specific drugs. By integrating genomic alterations with drug response data, the dataset supports the discovery of mechanisms driving drug action and resistance. Furthermore, GDSC1 aids in the development of predictive algorithms for personalized medicine, where treatments can be tailored to the genetic profile of a patient's tumor.

The dataset is also a resource for drug repurposing, as it allows researchers to identify new therapeutic indications for existing drugs based on their activity in genetically defined cell lines. Additionally, GDSC1 serves as a reference for benchmarking computational methods in drug sensitivity prediction and for training machine learning models to forecast therapeutic responses.

*Drugs Tested and Their Classes*

The GDSC1 dataset includes data on the response of cancer cell lines to 265 compounds representing a wide array of therapeutic classes. These drugs encompass:

- Cytotoxic agents: Traditional chemotherapy drugs that target rapidly dividing cells.
- Targeted therapies: Compounds designed to inhibit specific oncogenic pathways, including kinase inhibitors targeting EGFR, HER2, BRAF, MEK, and PI3K.
- Epigenetic modulators: Agents targeting histone deacetylases (HDACs) and DNA methyltransferases (DNMTs).
- Hormonal therapies: Drugs like anti-estrogens and anti-androgens used in hormone-dependent cancers.
- Immunomodulators: Although limited in scope in GDSC1, some early-stage immune-targeted agents are included.

The diverse drug panel reflects both approved therapeutics and experimental compounds under clinical investigation, ensuring the dataset's relevance for translational cancer research.

*Gene Expression Platform*

Gene expression data in the GDSC1 dataset were obtained using Affymetrix GeneChip microarrays, a widely used platform for high-throughput transcriptomic profiling. These arrays enabled the quantification of gene expression levels across tens of thousands of transcripts, providing a comprehensive view of the transcriptomic landscape of the cell lines. This dataset complements other genomic features, such as mutations and CNVs, facilitating integrative analyses to uncover relationships between gene expression patterns and drug responses.

*Impact and Applications*

The gene expression data in the GDSC1 dataset were used to calculate the ImmunoScore for each cell line that is part of the data set. Next, the IC50 values of the drugs that were tested on the GDSC1 bladder cancer cell lines (18 cell lines in total) were tested in correlation analysis to the log2 value of the ImmunoScore or the PDE4D gene (see correlation figures). The tested FGFRi were: AZD4547, FGFR_3831, PD173074.

The panel of 18 tested bladder cancer cell lines were: 5637, RT4, SW780, TCCSUP, T24, UM-UC-3, DSH1, J82, LB831-BLC, 647-V, 639-V, HT-1197, HT-1376, KU-19-19, RT-112, SW1710, BFTC-905, and CAL-29

NOTE: for some experiments only 16 or 17 of the 18 cell lines could be included into the final regression analysis.

The results are depicted in the appended figures. In brief, there is a clear correlation between immunoscore and IC50, meaning that higher Immunoscore corresponds to a higher IC50 value and vice versa, indicating that a low Immunoscore predicts better treatment response to FGFR inhibitor mediated therapy (and vice versa). In addition, a high PDE4D expression level corresponds to a low IC50 value and vice versa, meaning that a high PDE4D expression level predicts better treatment response to FGFR inhibitor mediated therapy (and vice versa).

**Claims**

1. A Fibroblast Growth Factor Receptor (FGFR) inhibitor for use in the treatment of bladder cancer in a subject in need thereof, the use comprising
determining in a bladder cancer sample obtained from the subject the expression level of PDE4D, and administering the FGFR inhibitor to the subject if the expression levels is above a predetermined threshold.

2. A Fibroblast Growth Factor Receptor (FGFR) inhibitor for use in the treatment of bladder cancer in a subject in need thereof, the use comprising
determining in a bladder cancer sample obtained from the subject the expression levels of three or more genes selected from APOBEC3A, CIAO1, DDX58, DHX9, IFI16, IFIH1, LRRFIP1, OAS1, ZBP1, EZR, and PDE4D, and calculating a FGFR inhibitor treatment score based on the three or more expression levels, and administering the FGFR inhibitor to the subject if the FGFR inhibitor treatment score is below a predetermined threshold.

3. FGFR inhibitor for use according to claim 2, wherein the FGFR inhibitor treatment score is calculated using the formula:

$$\text{FGFRi\_Score} = a * [\text{gene\_A}] + b * [\text{gene\_B}] + c * [\text{gene\_C}] + d$$

wherein a, b, c, and d are constants, and [gene_A], [gen_B], and [gene_C] are each a numerical values representing the expression levels of the three or more selected genes, and FGFRi_Score is the FGFR inhibitor treatment score.

4. FGFR inhibitor for use according to any one of the preceding claims, wherein the FGFR inhibitor is selected from erdafitinib, pemigatinib, futibatinib, infigratinib, resigratinib (KIN-3248), surufatinib, lucitanib(E3810), dovitinib(TKI258), lenvatinib (E7080), ponatinib (AP24534), regorafenib (BAY 73-4506), cediranib (AZD2171), intedanib (BIBF 1120), Fexagratinib (AZD4547), PD173074, FGFR_3831, and brivanib (BMS-540215).

5. FGFR inhibitor for use according to any one of the preceding claims, wherein the threshold is predetermined in a cohort of bladder cancer samples by correlating the PDE4D expression level or the FGFR inhibitor treatment score to the IC50 of the FGFR inhibitor.

6. FGFR inhibitor for use according to any one of the preceding claims, wherein the bladder cancer sample is a sample comprising at least one bladder cancer cell, preferably wherein the sample is biopsy, or tissue resection of the bladder or a metastasis thereof, or a liquid biopsy.

7. A computer implemented method of predicting effectiveness of treating a subject with bladder cancer with an FGFR inhibitor, the method comprising

receiving the expression level of PDE4D determined in a bladder cancer sample obtained from the subject, and predicting the effectiveness of treating the subject with a FGFR inhibitor based on the PDE4D expression level.

8. A computer implemented method of predicting effectiveness of treating a subject with bladder cancer with an FGFR inhibitor, the method comprising

receiving the expression levels of three or more genes selected from APOBEC3A, CIAO1, DDX58, DHX9, IFI16, IFIH1, LRRFIP1, OAS1, ZBP1, EZR, and PDE4D determined in a bladder cancer sample obtained from the subject, and
calculating a FGFR inhibitor treatment score based on the three or more expression levels, and predicting the effectiveness of treating the subject with a FGFR inhibitor based on the FGFR inhibitor treatment score.

9. Computer implemented method according to claim 7 or 8, wherein the bladder cancer sample is a sample comprising at least one bladder cancer cell, preferably wherein the sample is biopsy, or tissue resection of the bladder or a metastasis thereof, or a liquid biopsy.

10. Computer implemented method according to claim 8, wherein the FGFR inhibitor treatment score is calculated using the formula:

$$\text{FGFRi\_Score} = a * [\text{gene\_A}] + b * [\text{gene\_B}] + c * [\text{gene\_C}] + d$$

wherein a, b, c, and d are constants, and [gene_A], [gen_B], and [gene_C] are each a numerical values representing the expression levels of the three or more selected genes, and FGFRi_Score is the FGFR inhibitor treatment score.

11. Computer implemented method according to any one of claims 7 to 10, wherein the FGFR inhibitor is selected from erdafitinib, pemigatinib, futibatinib, infigratinib, resigratinib (KIN-3248), surufatinib, lucitanib(E3810), dovitinib(TKI258), lenvatinib (E7080), ponatinib (AP24534), regorafenib (BAY 73-4506), cediranib (AZD2171), intedanib (BIBF 1120), Fexagratinib (AZD4547), PD173074, FGFR_3831, and brivanib (BMS-540215).

12. Computer implemented method according to any one of claims 7 to 11, wherein the threshold is predetermined in a cohort of bladder cancer samples by correlating the PDE4D expression level or the FGFR inhibitor treatment score to the IC50 of the FGFR inhibitor.

13. Computer implemented method according to any one of claims 7 to 12, wherein the method is used to provide a treatment recommendation to the subject or a caregiver.

14. A kit of parts comprising a FGFR inhibitor and means for determining the expression levels of PDE4D and/or three or more genes selected from APOBEC3A, CIAO1, DDX58, DHX9, IFI16, IFIH1, LRRFIP1, OAS1, ZBP1, EZR, and PDE4D in a sample.

15. Use of a kit to predict effectiveness of treating a subject with bladder cancer with an FGFR inhibitor, the kit comprising means for determining the expression levels of PDE4D and/or three or more genes selected from APOBEC3A, CIAO1, DDX58, DHX9, IFI16, IFIH1, LRRFIP1, OAS1, ZBP1, EZR, and PDE4D in a sample, wherein the use comprises determining the expression levels of PDE4D and/or three or more genes selected from APOBEC3A, CIAO1, DDX58, DHX9, IFI16, IFIH1, LRRFIP1, OAS1, ZBP1, EZR, and PDE4D in a bladder cancer sample from a subject with bladder cancer, and predicting the effectiveness of treating the subject with bladder cancer with an FGFR inhibitor based on the PDE4D expression level and/or the expression levels of the three or more genes selected from APOBEC3A, CIAO1, DDX58, DHX9, IFI16, IFIH1, LRRFIP1, OAS1, ZBP1, EZR, and PDE4D.

**Fig. 1**

**Fig. 2**

Fig. 3

Fig. 4

y = 3.648 + -0.111 x
n = 18
r = 0.58; P = 0.012

**Fig. 5**

y = 3.679 + -0.126 x
n = 18
r = 0.60; P = 0.008

**Fig. 6**

Europäisches
Patentamt

European
Patent Office

Office européen
des brevets

## EUROPEAN SEARCH REPORT

**Application Number**

EP 24 21 9335

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/166979 A1 (THOMAS ROMAN K [DE] ET AL) 15 June 2017 (2017-06-15) * the whole document * * para. 6-10, 28-34; claims * | 1-15 | INV. C12Q1/6886 |
| Y | ZENGIN ZEYNEP B ET AL: "Targeted therapies: Expanding the role of FGFR3 inhibition in urothelial carcinoma", UROLOGIC ONCOLOGY: SEMINARS AND ORIGINAL INVESTIGATIONS, ELSEVIER, AMSTERDAM, NL, vol. 40, no. 2, 26 November 2021 (2021-11-26), pages 25-36, XP086943468, ISSN: 1078-1439, DOI: 10.1016/J.UROLONC.2021.10.003 [retrieved on 2021-11-26] * the whole document * * Tab. 1-3, see 1.3.3 Rogaratinib (BAY 1163877), 1.3.9 AZD 4547 * | 1-15 | |
| Y | WO 2021/262696 A2 (GENECENTRIC THERAPEUTICS INC [US] ET AL.) 30 December 2021 (2021-12-30) * the whole document * * Para. 7-9; claims * | 1-15 | **TECHNICAL FIELDS SEARCHED (IPC)** C12Q |
| X | CN 115 125 303 A (UNIV PEKING FIRST HOSPITAL) 30 September 2022 (2022-09-30) | 14 | |
| Y | * the whole document * * Para. 3-6, 36-51; claims; Example 3 * | 1-13,15 | |
| X | US 2009/048266 A1 (HEISE CARLA [US] ET AL) 19 February 2009 (2009-02-19) | 14 | |
| Y | * the whole document * * Para. 60, 62, 66, 71 -74; Table I; claims 10, 63 * | 1-13,15 | |

-/--

The present search report has been drawn up for all claims

| Place of search | Date of completion of the search | Examiner |
|---|---|---|
| Munich | 16 May 2025 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 24 21 9335 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2024/061634 A1 (KONINKLIJKE PHILIPS NV [NL]) 28 March 2024 (2024-03-28) * the whole document * * Claims * ----- | 1-15 | |
| A | QIANG ZHE ET AL: "Inhibition of TPL2 by interferon-[alpha] suppresses bladder cancer through activation of PDE4D", JOURNAL OF EXPERIMENTAL & CLINICAL CANCER RESEARCH, vol. 37, no. 1, 27 November 2018 (2018-11-27), XP055783668, DOI: 10.1186/s13046-018-0971-4 Retrieved from the Internet: URL:http://link.springer.com/content/pdf/1 0.1186/s13046-018-0971-4.pdf> * the whole document * * Abstract; Fig. 6 * ----- | 1-15 | |
| A | EP 3 960 877 A1 (KONINKLIJKE PHILIPS NV [NL]) 2 March 2022 (2022-03-02) * the whole document * * Claims * ----- | 1-15 | TECHNICAL FIELDS SEARCHED (IPC) |

The present search report has been drawn up for all claims

| Place of search | Date of completion of the search | Examiner |
|---|---|---|
| Munich | 16 May 2025 | Sauer, Tincuta |

EPO FORM 1503 03.82 (P04C01)

page 2 of 2

## ANNEX TO THE EUROPEAN SEARCH REPORT
## ON EUROPEAN PATENT APPLICATION NO.

EP 24 21 9335

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | | | Publication date | Patent family member(s) | | Publication date |
|---|---|---|---|---|---|---|
| US 2017166979 | A1 | | 15-06-2017 | CA | 2862530 A1 | 20-11-2015 |
| | | | | US | 2015335643 A1 | 26-11-2015 |
| | | | | US | 2017166979 A1 | 15-06-2017 |
| WO 2021262696 | A2 | | 30-12-2021 | CA | 3188105 A1 | 30-12-2021 |
| | | | | EP | 4262984 A2 | 25-10-2023 |
| | | | | US | 2023243813 A1 | 03-08-2023 |
| | | | | WO | 2021262696 A2 | 30-12-2021 |
| CN 115125303 | A | | 30-09-2022 | NONE | | |
| US 2009048266 | A1 | | 19-02-2009 | AU | 2006321602 A1 | 14-06-2007 |
| | | | | BR | PI0619548 A2 | 04-10-2011 |
| | | | | CA | 2630597 A1 | 14-06-2007 |
| | | | | CN | 101326292 A | 17-12-2008 |
| | | | | EP | 1960547 A2 | 27-08-2008 |
| | | | | EP | 2546359 A1 | 16-01-2013 |
| | | | | JP | 2009523410 A | 25-06-2009 |
| | | | | KR | 20080080525 A | 04-09-2008 |
| | | | | US | 2009048266 A1 | 19-02-2009 |
| | | | | US | 2012149714 A1 | 14-06-2012 |
| | | | | WO | 2007067968 A2 | 14-06-2007 |
| WO 2024061634 | A1 | | 28-03-2024 | EP | 4343003 A1 | 27-03-2024 |
| | | | | WO | 2024061634 A1 | 28-03-2024 |
| EP 3960877 | A1 | | 02-03-2022 | CN | 116783310 A | 19-09-2023 |
| | | | | EP | 3960877 A1 | 02-03-2022 |
| | | | | EP | 4204589 A1 | 05-07-2023 |
| | | | | JP | 2023538944 A | 12-09-2023 |
| | | | | US | 2023407402 A1 | 21-12-2023 |
| | | | | WO | 2022043299 A1 | 03-03-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning. A Laboratory Manual. Cold Spring Harbor Laboratory Press, 1989
- **AUSUBEL et al.** Current Protocols in Molecular Biology. John Wiley & Sons, 1987
- Methods in Enzymology. Academic Press
- Computational Molecular Biology. Oxford University Press, 1988
- Biocomputing: Informatics And Genome Projects. Academic Press, 1993
- Computer Analysis Of Sequence Data. Humana Press, 1994, vol. I
- **VON HEINJE, G.** Sequence Analysis In Molecular Biology. Academic Press, 1987
- Sequence Analysis Primer. M Stockton Press, 1991
- **CARILLO, H. ; LIPTON, D.** *SIAM J. Applied Math*, 1988, vol. 48, 1073
- Guide To Huge Computers. Academic Press, 1994
- **CARILLO, H. ; LIPTON, D.** *Siam J. Applied Math*, 1988, vol. 48, 1073
- **DEVEREUX, J. et al.** *Nucleic Acids Research*, 1984, vol. 12 (1), 387
- **ATSCHUL, S. F. et al.** *J. Molec. Biol.*, 1990